# EUROPEAN PATENT SPECIFICATION

(11) **EP 0 937 465 B1**
(45) Date of publication and mention of the grant of the patent: **17.08.2005**
(21) Application number: 99103020.6
(22) Date of filing: 16.02.1999
(51) Int. Cl.: A61L 2/06

(54) **Process and apparatus for steam sterilization, in particular for dental uses**
Verfahren und Gerät zur Dampfsterilisation, insbesondere für dentale Zwecke
Procédé et appareil de stérilisation à vapeur particulièrement pour usage dentaire

(30) Priority: 20.02.1998 IT MI980329
(43) Date of publication of application: 25.08.1999
(73) Proprietor: M.O.COM. S.r.L., 20124 Milano (IT)
(72) Inventor: Piazzalunga, Gianfranco, 20080 Casarile (Milano) (IT); Villani, Yuri, 20090 Trezzano S/Naviglio (IT); Tosi, Daniele, 27018 Vidigulfo (Pavia) (IT)
(74) Representative: Lunati, Vittoriano, Dr.Ing.

(56) References cited:
- EP-A2- 0 429 960

## Description

The invention relates to a process and an apparatus for steam sterilization, in particular for dental uses, of the type pointed out in the preamble of the claims and comprising an autoclave defining a sterilization chamber, a duct for feeding water to said chamber, steam generating means associated with said autoclave, and a discharge circuit from said chamber comprising a condenser and at least one suction means, and wherein the sterilization chamber is brought to the sterilization pressure and temperature and is then evacuated by discharging condensate and steam therefrom.

It is known that apparatuses of this kind comprise an autoclave, defining a sterilization chamber in which objects, in particular dental instruments, to be sterilized or disinfected are placed. Distilled water is fed to the chamber from a feed tank through a feeding duct in which a pump is arranged, and the feed water is heated to form steam at such a pressure and temperature that a sterilizing action is performed on the objects contained in the chamber.

Also provided is a discharge duct, extending between the autoclave and a discharge tank and in which there is a condenser consisting of a coil placed in the water feeding tank itself and a vacuum pump or other suction means for sucking air, condensate and steam from the sterilization chamber so that the chamber is evacuated and a vacuum is created in the chamber itself under given steps of the sterilization cycle.

A valve system makes the feeding and discharge ducts become operating during the sterilization cycle, together with the respective pumps, according to preestablished sterilization programs.

In these apparatuses the vacuum pump must necessarily suck, in addition to air and steam, also condensate and water mixed with steam, and this may lead to pump fatigue with a consequent loss of efficiency of said pump.

Occurrence of this drawback is increasingly more possible as the efficiency in eliminating all traces of condensate is wished to be increased. As a result, vacuum pumps of appropriate structure and power are required, which on the other hand increases costs for apparatuses.

In some known apparatuses provision is made for a second condenser in addition to the above mentioned condenser present in the water feeding tank; said second condenser consists of a chamber having appropriate condensation surfaces and is disposed in series to the vacuum pump, upstream of the latter.

In this way, fluid being evacuated from the sterilization chamber is further condensed and the condensate liquid is collected at the chamber bottom so as to greatly reduce the amount of water to be conveyed by the vacuum pump and enable arrangement of a less expensive pump, in particular a diaphragm pump.

This solution however can bring to a dangerous heating of the second condenser through which very hot condensate can still pass, and this may lead to loss of efficiency of the condenser itself, in that heating of its walls reduces the condensing capability thereof.

It may happen therefore that the vacuum pump is still obliged to suck very important amounts of condensate from the sterilization chamber and consequently important amounts of water, so that a pump of small sizes and power cannot be employed.

It will be also recognized that if the apparatus elements are not in a condition to operate with high efficiency, it is not even possible to evacuate all residual condensate and steam, unless the duration of the sterilization cycle is greatly increased.

Under this situation, the technical task of the invention is to substantially obviate the above mentioned drawbacks.

Within the scope of this technical task it is an important aim of the invention to conceive a process and an apparatus enabling the overall sterilization efficiency to be increased while at the same time reducing duration of the sterilization cycle.

It is a further aim of the invention to conceive a sterilization process and the relative sterilization apparatus enabling condensate to be efficiently eliminated without employing an expensive vacuum pump and without any risk of pump fatigue, and also without increasing times for evacuating the sterilization chamber.

A still further aim of the invention is to conceive a sterilization process and apparatus ensuring an optimal drying even of porous and bulky objects that are generally more difficult to be treated.

The above specified technical task and aims are achieved by a process and an apparatus for steam sterilization, in particular for dental uses, as claimed in the appended claims 1 and 8, respectively.

Preferred embodiments are then highlighted in the appended sub-claims.

Details and advantages of the invention will be more apparent from the detailed description of two preferred embodiments given with reference to the accompanying drawings, in which:
- Fig. 1 is a hydraulic diagram of a first embodiment of an apparatus in accordance with the invention;
- Fig. 2 is a diagram of the pressure course in the sterilization chamber during a sterilization cycle of the fractional vacuum type;
- Figs. 3, 4, 5 show simplified hydraulic diagrams of the apparatus where the fluid paths of travel during some steps of the process of the invention are highlighted;
- Fig. 6 shows the hydraulic diagram of another embodiment of an apparatus of the invention;
- Fig. 7 shows a diagram of the pressure course during another sterilization cycle to be executed with the apparatus in Fig. 6; and
- Figs. 8, 9, 10 diagrammatically show the fluid paths of travel during some steps of the cycle in Fig. 7.

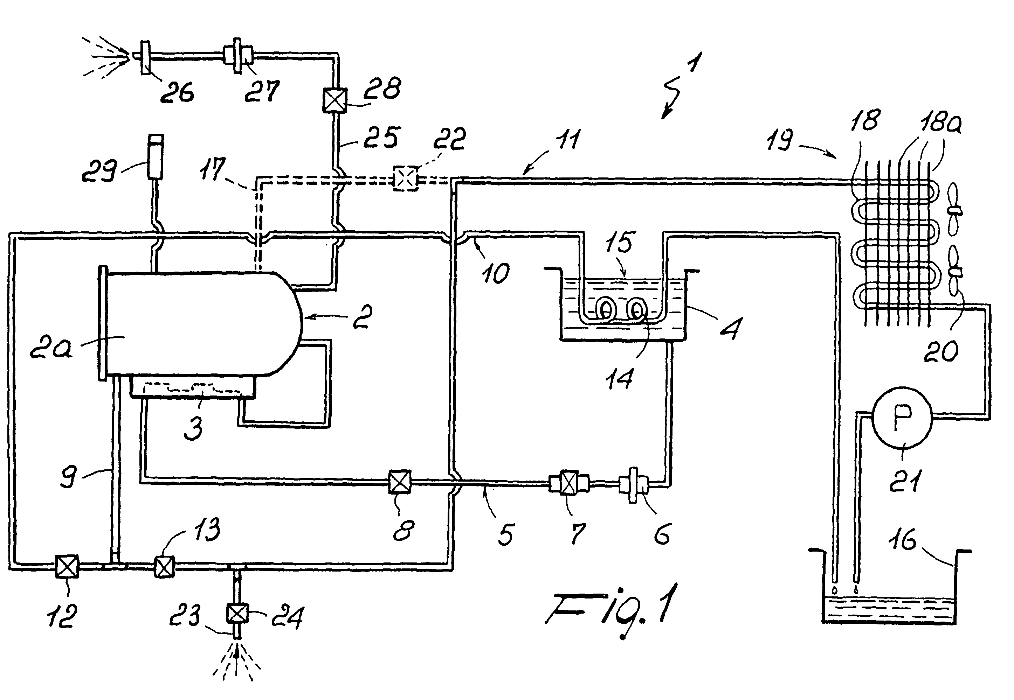

With reference to Figs. 1-5, an apparatus 1 for steam sterilization according to the invention comprises, in a manner known per se, an autoclave 2 defining a sterilization chamber 2a in which objects to be sterilized, dental instruments or tools for example, are placed. Associated with the autoclave 2 is for example a steam generator 3 of known type.

The sterilization chamber 2a is connected with a water feeding tank 4 through a feeding duct 5, within which a filter 6, a feed pump 7 and a shutoff valve 8 are disposed. As shown, part of duct 5 passes through the steam generator 3 before reaching chamber 2a. Tank 4 is filled with distilled water.

Starting from bottom of chamber 2a is an outlet duct 9, branching out in a first discharge duct 10 and a second discharge duct 11. Ducts 10 and 11 are controlled by respective valves 12 and 13. The discharge duct 10 for example, passes in the form of a coil 14 at the inside of the feed tank 4, forming a first condenser 15 with the water contained therein, and terminates into a discharge tank 16.

An auxiliary discharge duct 17 directly opening into the second discharge duct 11 may be provided starting from the upper part of chamber 2a. Fitted in duct 11 is a heat exchanger, consisting in particular of a coil 18 having cooling fins 18a, forming a second condenser 19 which is air cooled, in particular by means of fans 20.

From condenser 19, duct 11 reaches the discharge tank 16 and in this length of duct 11 there is a vacuum pump or another suction means 21, in particular a diaphragm pump. A shutoff valve 22 is fitted in the auxiliary duct 17 connected to the autoclave 2 top.

The second discharge duct 11 is connected to an air feeding duct 23, a shutoff valve 24 being interposed therebetween.

In addition, a sterile-air feed duct 25 opens into the upper part of the sterilization chamber 2a, in which duct a bacteriologic filter 26, a common filter 27 and a shutoff valve 28 are disposed. Denoted by 29 is a safety valve of known type, associated with the autoclave 2.

From the above description it will be clear that, unlike known apparatuses, in apparatus 1 in accordance with the invention two distinct discharge circuits are actually provided, i.e. a circuit comprising the discharge duct 10 forming the first condenser 15 and freely discharging into the discharge tank 16, and a circuit comprising the discharge duct 11 passing through the second condenser 19 and in which the vacuum pump 21 is fitted.

Actuation of the feeding and discharge circuits by the respective valves and pumps is controlled by a programmed central unit for carrying out the sterilization cycle in a completely automatic manner.

A sterilization process to be put into practice by the above described apparatus, a fractional vacuum process for example, may provide the following steps.

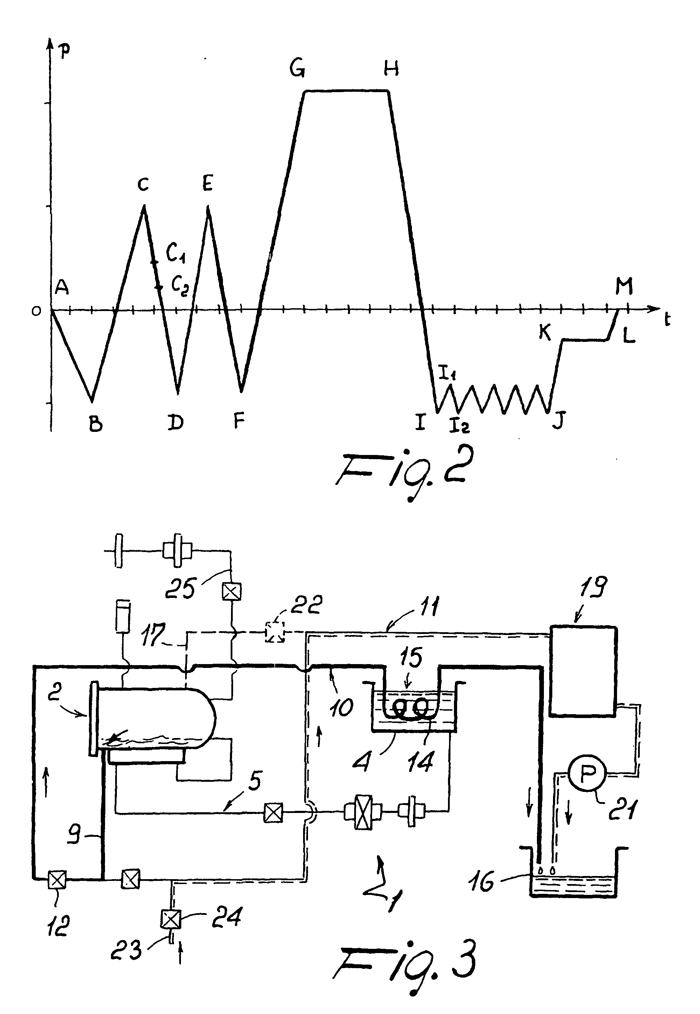

In a first step, shown by line A-B in the diagram in Fig. 2, air is sucked from the sterilization chamber 2a by the vacuum pump 21 through the second discharge duct 11, passing through the second condenser 19.

Valve 13 is open (and valve 22 too, if the auxiliary duct 17 is present) and valves 12 and 24 are closed, so that air is sucked through ducts 9 and 11 (and through the auxiliary duct 17, if it is present). During this step vacuum is created also in the portion of the feeding duct 5 placed between valve 8 and the sterilization chamber 2a. Vacuum can reach -0.90 bar, for example.

In the subsequent step (line B-C of the diagram in Fig. 2) the vacuum pump 21 is kept deactivated and the valve 8 and feed pump 7 are activated; pump 7 draws water from the feed tank 4 and pushes it, through valve 8 which is open, into the steam generator 3 which produces steam flowing into the sterilization chamber 2a, thereby increasing pressure in said chamber until 1.00 bar for example.

At this point, the step of discharging condensate and steam from chamber 2a begins, which step is represented by the initial length of line C-D in the diagram in Fig. 2.

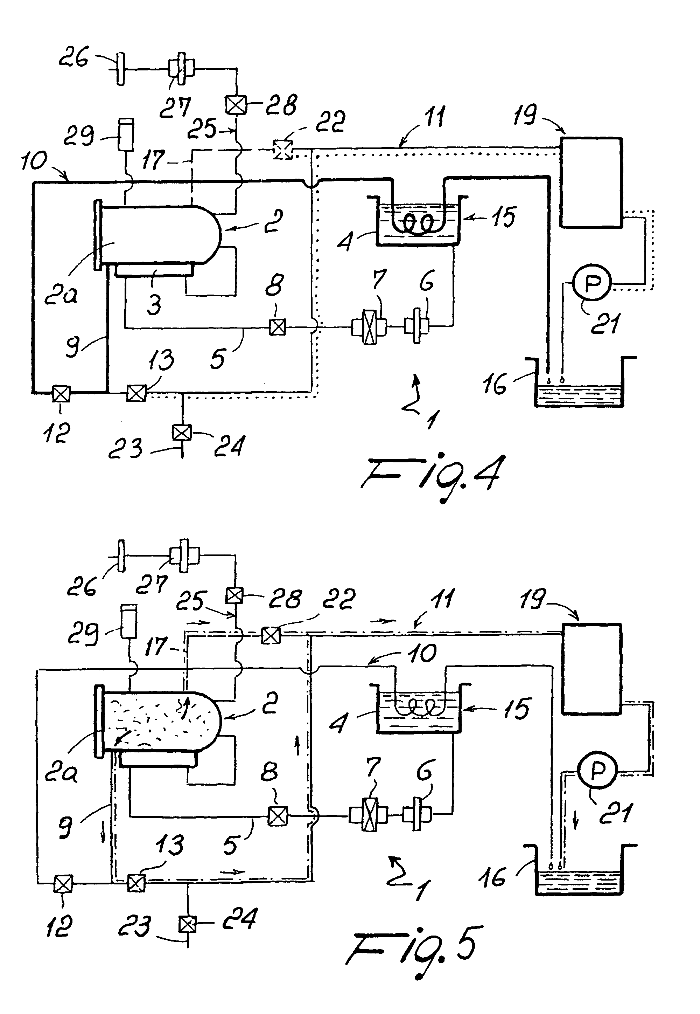

Condensate is discharged from the bottom of chamber 2a through the outlet duct 9 and the first discharge duct 10, as shown by a thick solid line in Fig. 3, valve 12 being open and valve 13 closed. Also closed is valve 22, if duct 17 is provided.

Discharging of the condensate takes place due to pressure in chamber 2a. In this step the hot condensate and steam are cooled in the first condenser 15, transferring part of their heat to the feed water that will then reach the steam generator 3 at a higher temperature, so that energy needs for reaching the wished pressure and temperature conditions in chamber 2a for subsequent sterilization will be reduced.

It is to note that in this step there is no passage of condensate either through the second condenser 19 or through the vacuum pump 21. This prevents condenser 19 from being dangerously heated and avoids a heavy activity of pump 21.

Simultaneously with condensate discharging, valve 24 is opened and the vacuum pump 21 is actuated so that it sucks air from the feeding duct 23 along the discharge duct 11, as shown in chain line in Fig. 3.

Air flowing in this circuit carries out cleaning of the circuit and in particular of condenser 19, by eliminating the residual condensate and enabling condenser 19 to be afterwards in its best efficiency conditions, i.e. cool and dry, for performing its function.

Condensate discharging and cleaning take place until point C1 of line C-D in the diagram in Fig. 2, until a pressure of 0.30 bar for example is reached. It is to note that the cleaning step takes place simultaneously with the condensate discharging, due to hydraulic separation of the two discharge ducts 10 and 11, so that the cleaning step does not involve any increase in the duration of the sterilization cycle.

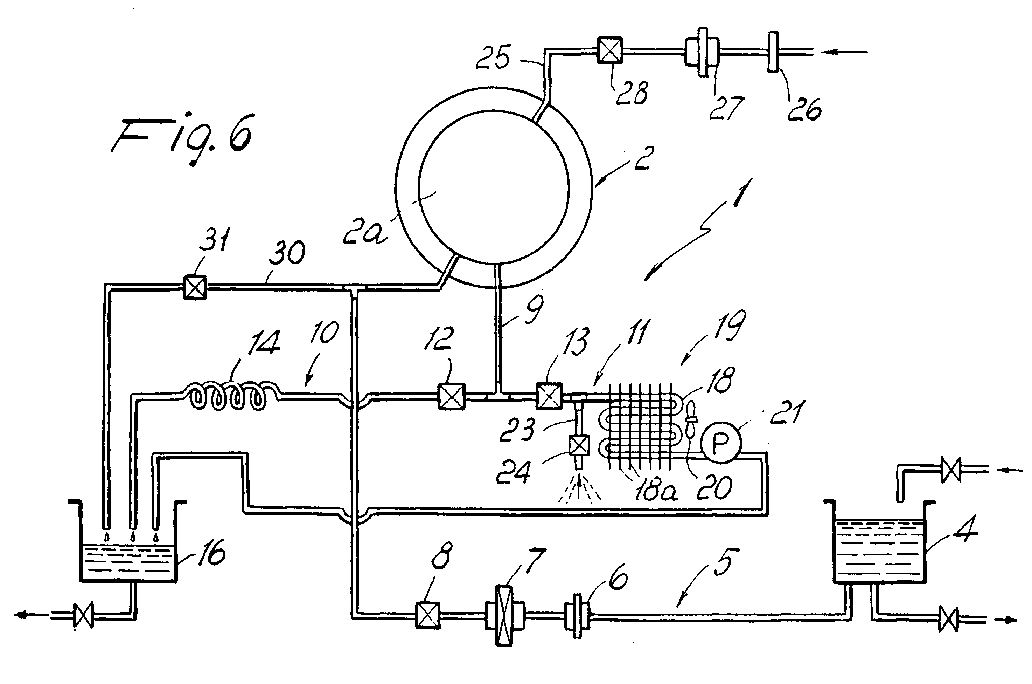

Valve 24 can now be closed. Since the vacuum pump 21 is still operating, the vacuum is created in the discharge duct 11, as shown in dotted line in Fig. 4, thereby producing a high vacuum value, due to the small volume of the concerned circuit.

Chamber 2a goes on discharging into duct 10, through valve 12. This vacuum step lasts until point C2 of the diagram in Fig. 2, until 0.20 bar for example. Now valve 12 is closed and valve 13 is opened, enabling pump 21 to suck steam from chamber 2a through the outlet duct 9 and discharge duct 11.

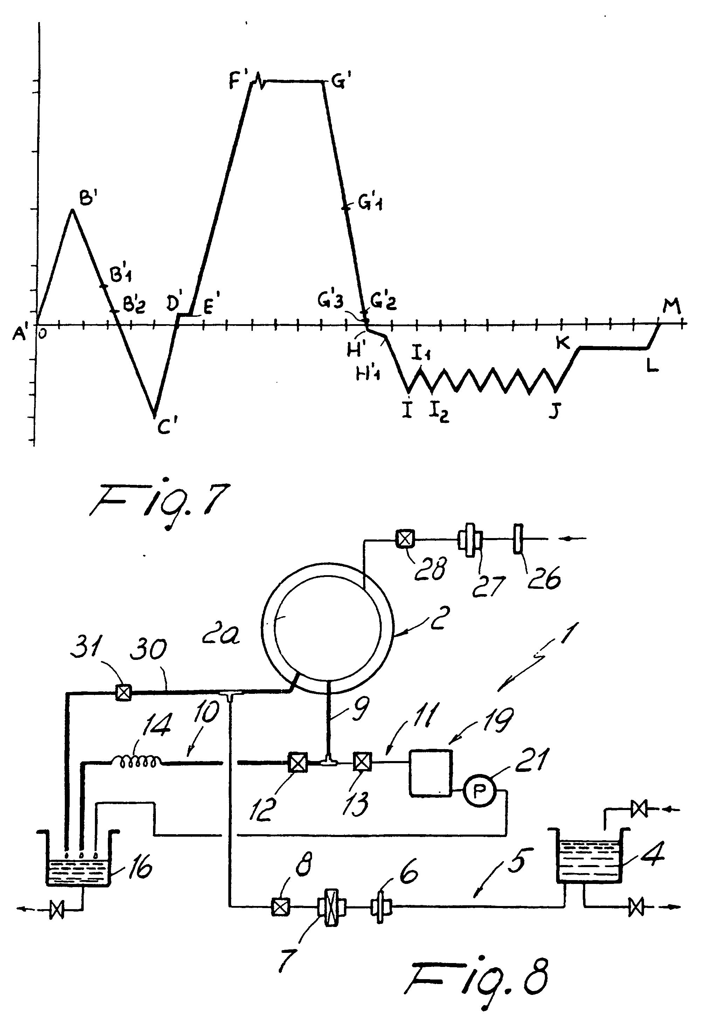

If the auxiliary duct 17 is present, valve 22 too is opened, thereby achieving an efficient steam-evacuation action simultaneously from two points of chamber 2a, as shown in chain dot line in Fig. 5. Action of pump 21 is maintained over the whole line C2-D in the diagram in Fig. 2, gradually bringing chamber 2a back to the vacuum, until a value of -0.80 bar, for example.

It is to note that during this step condenser 19 is capable of providing excellent performance, as it has been only just drained and cooled. Since much steam and little air are present, condenser 19 is capable of quickly condensing steam again, thereby reducing volume of the fluid to be sucked.

The condensate being formed is thus immediately evacuated by the vacuum pump 21. During this step the vacuum is also created in the plant portion between valve 8 which is closed and chamber 2a.

At this point, as viewed from diagram in Fig. 2, either a step of pressurizing chamber 2a can be repeated and then a step of evacuating condensate and steam (lines D-E and E-F in the diagram), or chamber 2a can be brought to the sterilization pressure and temperature for the true sterilization process, as denoted by line F-G in the diagram.

Obviously, the pressurization and evacuation steps in question could be repeated different times.

It is to note that also in each subsequent evacuation and vacuum steps (descending line in the diagram), the cleaning step and the step of previously creating the vacuum in duct 11, described with reference to lines C-C1 and C1-C2 in the diagram in Fig. 2, are provided.

The step leading to achievement of the sterilization conditions according to line F-G in the diagram takes place in the same manner as for line B-C, keeping valve 8 open and all other valves closed.

The true sterilization step identified by line G-H in the diagram in Fig. 2, involves keeping temperature (and consequently pressure) in the sterilization chamber 2a to preestablished values. These conditions are automatically maintained in known manner.

When the sterilization step is over, chamber 2a is evacuated as already described for line C-D in the diagram, i.e. first steam and condensate are discharged through ducts 9 and 10 and the second discharge duct 11 is simultaneously cleaned, then intervention of these ducts and of condenser 19 under optimal conditions is caused for reaching the vacuum in chamber 2a, of a value in the order of -0.80 bar for example, which will bring about the already described advantages. Thus point I in the diagram in Fig. 2 is reached.

Now a drying step is started, and it can be carried out by extracting the residual steam from the sterilization chamber 2a by means of the vacuum pump 21 and keeping the vacuum in chamber 2a in an oscillating state between preestablished maximum and minimum values for example, as shown between points I and J in the diagram.

In other words, repetition of individual steps of admitting sterile air to chamber 2a from duct 25 (line I-I1) and extracting steam mixed with air (line I1-I2) can be provided.

Extraction of the residual steam is carried out by pump 21 along the discharge duct 11 after valve 13 has been opened, and also through the auxiliary duct 17, if provided, after opening valve 22. A vacuum of -0.90 bar can be achieved, for example.

Admission of sterile air takes place by opening valve 28 and keeping the vacuum pump 21 in operation. Pressure can rise to 0.80 bar for example. At this point it is sufficient to close valve 28 again, so that pressure goes down, and so on until point J.

This step of intermittently admitting sterile air to chamber 2a and continuously drawing the residual steam from the chamber itself enables an optimal drying of the objects to be obtained, in particular when porous and bulky objects are concerned.

When this step has been completed, ventilation of the sterilization chamber 2a is carried out, by admitting sterile air from duct 25 and sucking fluid from chamber 2a through ducts 9 and 11 (and possibly 17), valves 28 and 13 (and possibly 22) being open.

Pressure first reaches point K and becomes stable on a negative value depending both on the delivery of pump 21 and on the conformation of the relative circuits, as well as on the conformation of duct 25 and the configuration and passage section of valve 28.

When point L has been reached, pressure within the sterilization chamber 2a is brought to the atmospheric pressure value, keeping valve 28 open, which valve goes on admitting sterile air to chamber 2a, and deactivating the vacuum pump 21.

The cycle ends at point M, when pressure within chamber 2a is comparable to the atmospheric pressure.

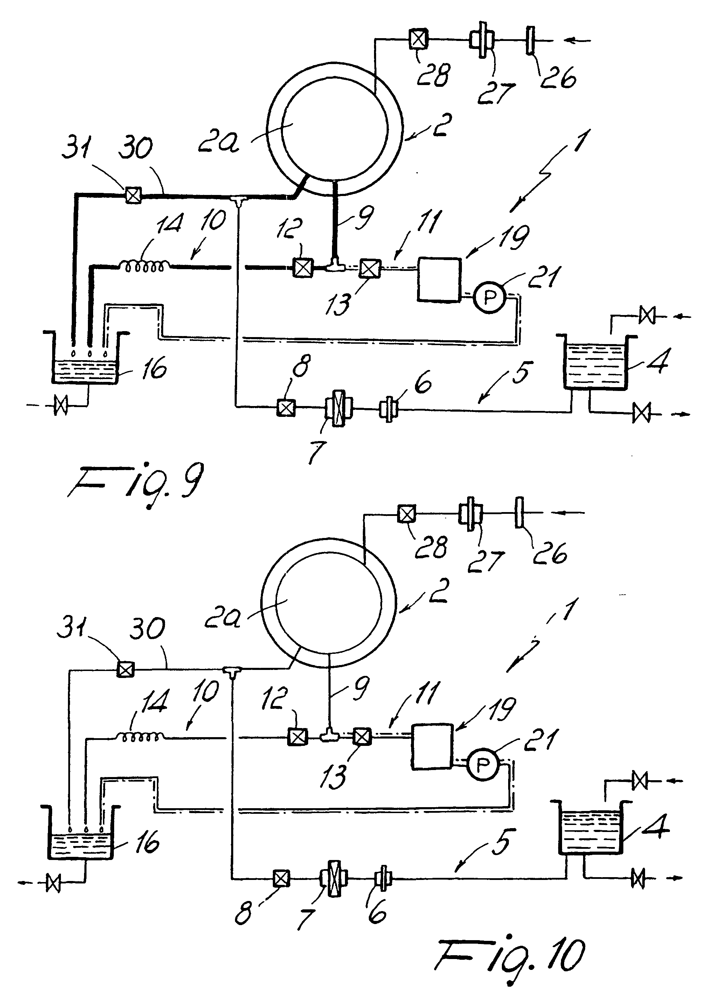

The apparatus shown in Figs. 6-10 is substantially similar to that in Figs. 1-5. Therefore similar or equivalent elements are identified by the same reference numerals.

This apparatus too comprises one feed duct 5 and two distinct discharge ducts 10 and 11. In the first discharge duct 10 the condensing coil 14 is placed in air, instead of being in the feed tank 4. The second discharge duct 11 comprises the shutoff valve 13, the condenser 19 consisting of a heat-exchanger 18 in air, possibly cooled by fans, and the vacuum pump 21, in the above order.

The possibility of admitting air to duct 11 downstream of valve 13 is provided, through the feeding duct 23 and the shutoff valve 24, for achieving the same aims as described for the embodiment in Figs. 1-5.

Likewise, admission of sterile air to chamber 2a is provided through the bacteriologic filter 26, common filter 27 and shutoff valve 28.

The apparatus of Figs. 6 to 10 in addition has a further discharge duct 30, within which a shutoff valve 31 is disposed and which directly opens into the discharge tank 16. This apparatus can be employed for carrying out various sterilization cycles, a single-vacuum cycle for example, as shown in the diagram in Fig. 7.

In this case first loading of water into chamber 2a is carried out through duct 5, and steam is produced therein, until a pressure of 1.00 bar (line A'-B' in the diagram in Fig. 7) is for example reached. Then a discharge step is carried out (line B'-B'1 in the diagram), by opening both valve 12 and valve 31 and thus causing operation of the further discharge duct 30, in addition to the discharge duct 10, as shown by thick solid lines in Fig. 8. Steam and condensate are discharged from chamber 2a, for example until a pressure of 0.30 bar is reached.

Advantageously, an air bleeding takes place too. During this step cleaning of the discharge duct 11 can be carried out by means of the air admitted from duct 23, as previously described in detail as regards the embodiment in Figs. 1-5.

At this point the second discharge duct 11 is activated as well, valve 13 being opened and the vacuum pump 21 being operated. This enables steam bleeding through the second condenser 19, as shown in chain dot line in Fig. 9, while steam and condensate bleeding through ducts 10 and 30 goes on. This step, represented by line B'1-B'2 in the diagram, can last until a pressure just higher than the atmospheric pressure is reached, for example.

Then a step of previous vacuum is started, in which the two discharge ducts 10 and 30 are completely deactivated through closure of valves 12 and 31 and the only discharge duct 11 is operated, as shown in Fig. 10.

This step, represented by line B'2-C' in the diagram, can last until a vacuum of -0.80 bar is reached. It involves suction of the residual air mixed with steam from chamber 2a.

As in the embodiment in Figs. 1-5, in this case too pump 21 has to suck the recondensed steam alone and therefore is not submitted to over-fatigue and can be a pump of small sizes and low costs. Since condenser 19 is not passed through by very hot condensate, a high efficiency of same can be maintained and consequently an efficient condensing action, which is advantageous for pump 21.

In addition, condenser 19 is of valid help to pump 21 also because in the presence of much steam and little air, as actually occurs in the apparatus of the invention, condenser 19 causes steam to be quickly recondensed, thereby reducing volume of the fluid to be sucked.

The subsequent step again involves loading of chamber 2a with water through the feeding duct 5, and generation of steam until a pressure value just higher than the atmospheric pressure, as identified by line C'-D' in the diagram in Fig. 7. The discharge ducts 10, 11 and 30 are completely deactivated and valves 12, 13 and 31 are closed.

A short air and steam bleeding step can be now provided by temporarily opening valve 31 (line D'-E' in the diagram). When valve 31 is closed again, pressure within chamber 2a begins to rise again until the sterilization pressure is reached, in particular 2.10 bars at 134°C and 1.08 bars at 121°C, as shown by line E'-F' in the diagram.

Along line F'-G' the thermodynamic parameters are balanced and temperature and pressure values are made stable to the said values, after a short settlement step for example, identified by the oscillations of the initial portion of line F'-G' in the diagram in Fig. 7.

Then the true sterilization process takes place in a manner known per se.

At the end of the sterilization process there is a first discharge step (line G'-G'1 in the diagram), in which the condensate is essentially evacuated from the sterilization chamber 2a through the first discharge duct 10 comprising the condensing coil 14. On the contrary, valve 31 keeps closed and the further discharge duct 30 keeps inactive, as well as the second discharge duct 11.

Due to the pressure action, the condensate collected at the bottom of chamber 2a is pushed into the first discharge duct 10 which is open, causing said condensate to completely flow out of the sterilization chamber 2a.

When a pressure of 1.00 bar is for example reached, valve 31 too is opened and the further discharge duct 30 is caused to intervene, whereas the second discharge duct 11 keeps inactive, since valve 13 is closed. This step is represented by line G'1-G'2 in the diagram and by figure 8. During this step there is a discharge of the residual condensate mixed with steam.

At this point the discharge duct 17 is activated by operation of valve 13 and vacuum pump 21; there is a discharge of steam and drying begins (Fig. 9). Pressure for example reaches a value just higher than the atmospheric pressure, then valves 12 and 31 are closed and the respective discharge ducts 10 and 30 are deactivated, whereas the vacuum pump 21 action goes on (Fig. 10), said pump sucking steam through condenser 19 until point G'3 is reached and subsequently point H' in the diagram in Fig. 7.

Now a short ventilation of chamber 2a can be executed, by briefly opening valve 28, so that the pressure drop in chamber 2a is slower (line H'-H'1 in the diagram), then drying under vacuum is started again when valve 28 is reclosed (line H'1-I in the diagram). From this moment on the cycle goes on as already described for example for the preceding embodiment, passing through points I1, I2, J, K, L until the end M of the sterilization cycle.

The invention achieves important advantages.

Due to the arrangement of two distinct discharge ducts 10 and 11, to be selectively activated independently of each other, the condensate can be evacuated almost completely without making it pass through the vacuum pump 21 and the condenser 19 disposed in series to the pump, thereby avoiding over-fatigue of the pump and a loss of efficiency of the condenser, due to a very detrimental heating thereof caused by a very hot condensate.

In fact, the presence of two distinct discharge ducts 10 and 11 enables condensate and steam to be first freely discharged through the first discharge duct 10 and intervention of the second discharge duct 11 through which steam is essentially discharged and the vacuum in chamber 2a is created, to be subsequently caused, when the condensate has been substantially discharged from chamber 2a and pressure in said chamber has dropped to a lower value than the sterilization one.

The efficient condensing action of condenser 19 and quick evacuation of chamber 2a enable times necessary for achieving the predetermined vacuum values to be greatly reduced, above all in the fractional vacuum process, thereby also reducing the overall duration of the sterilization cycle.

In addition, the dual discharge circuit enables the apparatus to be conveniently arranged for an efficient action of extracting, condensing and discharging steam, by previously removing the residual condensate both from the condenser and from the pump heads and also by creating a previous vacuum in duct 11 itself, so as to trigger extraction of steam from the sterilization chamber more easily, sucking also the last traces of condensate from the chamber in a more efficient manner.

This enables efficiency of the sterilization process to be improved without prolonging the overall time of the sterilization cycle, since said evacuation and pre-arrangement of the steam-discharge duct take place concurrently with discharging of the condensate through the other discharge duct or ducts.

An intermittent or pulse admission of air from duct 23 to duct 11 can be carried out during the suction step of the vacuum pump 21, by operation of valves 13 and 24 (and 22 if duct 17 is present) so that evacuation of the sterilisation chamber 2a is stopped during each pulse or period of air admission.

This enables the pump to be cleared from condensate clogging or condensate traces, at the pump heads for example, thereby always keeping the pump under optimal efficiency conditions.

Since the vacuum pump is no longer subjected to fatigue risks, it can be a less powerful and less expensive pump, and thereof of small sizes, which is advantageous for the apparatus costs.

In addition, by the circuit arrangement in accordance with the invention, passage of steam through coil 14 is greatly restricted and this is important when the coil is disposed in the feed tank 4, since this passage could cause the water temperature in the tank to rise too much, which would give rise to boiling of said water.

The alternate sequence of short steps of vacuum increase and reduction in the sterilization chamber and of short periods of sterile-air circulation in the chamber itself during drying, causes the residual condensate to be eliminated to a much greater degree from the objects and the chamber, in that the vacuum pump temporarily reaches curves of greater delivery, sucking the condensate formed during the drying process.

Modifications and variations to the invention are possible. Thus, for example, the step of creating the vacuum in duct 11 after the cleaning step carried out therein could be omitted.

The drying step could be carried out in a traditional manner, i.e. keeping the vacuum constant.

Instead of a single vacuum pump, two pumps could be for example arranged in parallel.

Instead of a vacuum pump, another suction means could be employed, a Venturi tube device for example.

Instead of the shutoff valves 12, 13, 22, 24, three-way valves could be provided, in particular three-way solenoid valves, disposed at the convergence points of the respective ducts and adapted to implement the desired fluid paths of travel each time.

The steam generator 3 could be eliminated and steam could be generated directly in chamber 2a for example, by means of electric resistors associated with the autoclave 2.

The discharge tank 16 could also be not incorporated into the sterilization apparatus.

Condenser 19 could be of a different type from that described and illustrated by way of example, it could be a condenser provided with Peltier cells, for example.

## Claims

1. A process for steam sterilization, in particular for dental uses, in which a sterilization chamber (2a) is brought to a sterilization pressure and temperature and is then evacuated by discharging condensate and steam from the chamber, and in which a condenser (19) and at least one suction means (21) are disposed in a discharge duct, **characterized in that** discharging is carried out through at least one first and one second discharge ducts (10, 11) to be activated in succession, and **in that** discharging through said second discharge duct (11), equipped with said condenser (19) and said at least one suction means (21), is carried out after pressure in said chamber (2a) has dropped to a value substantially lower than the sterilization pressure.

2. A process as claimed in claim 1, wherein activation of said second discharge duct (11) is carried out before deactivation of other discharge ducts.

3. A process as claimed in claim 1, wherein during at least one discharge step through said first discharge duct (10), circulation of air through said second discharge duct (11) is carried out.

4. A process as claimed in claim 3, wherein after said air-circulation step and before a discharge step taking place through said second discharge duct (11), a vacuum is created in said second discharge duct (11).

5. A process as claimed in claim 1, wherein after a sterilization step, a drying step is provided in which sterile air is admitted to said sterilization chamber (2a) in an intermittent manner, while steam is being extracted from said chamber (2a) in a continuous manner.

6. A process as claimed in claim 3, wherein several discharge steps from said sterilization chamber (2a) through said first discharge duct (10) are provided, and associated with each of said steps is said air-circulation step through said second discharge duct (11).

7. A process as claimed in claim 1, wherein during at least one suction step through said second discharge duct (11), an intermittent or pulse air admission to said second discharge duct (11) is carried out, evacuation of the sterilization chamber (2a) through said second discharge duct (11) being interrupted during each pulse or air-admission period.

8. An apparatus for steam sterilization, in particular for dental uses, comprising an autoclave (2) defining a sterilization chamber (2a), a duct (5) for feeding water to said chamber (2a), means (3) for steam generation associated with said autoclave (2), a discharge circuit from said chamber (2a) comprising a condenser (19) and at least one suction means (21) and controlling means for carrying out a sterilization cycle,
**characterized in that** said discharge circuit comprises at least one first and one second discharge ducts (10, 11) to be selectively activated independently of each other and in succession by said controlling means, said condenser (19) and said at least one suction means (21) being disposed in said second discharge duct (11).

9. An apparatus as claimed in claim 8, wherein an air feeding duct (23) is provided which is connected with said second discharge duct (11) and can be activated concurrently with activation of said first discharge duct (10).

10. An apparatus as claimed in claim 9, wherein an auxiliary discharge duct (17) is provided which connects said second discharge duct (11) to the upper portion of said sterilization chamber (2a), said auxiliary duct (17) being hydraulically separable from said second discharge duct (11).

11. An apparatus as claimed in claim 8, wherein starting from the bottom of said sterilization chamber (2a) there is an outlet duct (9) branching out into said first discharge duct (10) and into said second discharge duct (11), said outlet duct (9) being susceptible of selective hydraulic connection with said first discharge duct (10) and said second discharge duct (11), respectively.

12. An apparatus as claimed in claim 8, wherein said first discharge duct (10) has a coil-shaped length (14) disposed in said feed tank (4) so as to form a water-cooled condenser (15).

13. An apparatus as claimed in claim 8, wherein said condenser (19) disposed in said second discharge duct (11) is an air-cooled condenser.

14. An apparatus as claimed in claim 13, wherein said air-cooled condenser (19) consists of a coil (18) provided with cooling fins (18a).

15. An apparatus as claimed in claim 8, wherein said at least one suction means (21) is a vacuum pump, in particular a diaphragm pump.

16. An apparatus as claimed in claim 8, wherein at least one further discharge duct (30) is provided which comes out of said sterilization chamber (2a) and can be activated at least partly concurrently with activation of said first discharge duct (10).

## Patentansprüche

1. Ein Verfahren zur Dampfsterilisierung, und zwar insbesondere für die Anwendung im Dentalbereich, wobei eine Sterilisierungskammer (2a) jeweils auf Sterilisierungsdruck und -Temperatur gebracht und anschließend durch Auslassen des Kondensats und des Dampfs aus der Kammer entleert wird, und wobei ein Kondensator (19) und mindestens ein Saugelement (21) in einem Auslasskanal angeordnet sind, **dadurch gekennzeichnet, dass** die Entleerung durch mindestens einen ersten und einen zweiten jeweils aufeinander folgend zu aktivierenden Auslasskanal (10, 11) stattfindet, sowie **dadurch**, dass die Entleerung durch den genannten zweiten Auslasskanal (11), der mit dem genannten Kondensator (19) ausgestattet ist, und das genannte mindestens eine Saugelement (21) erfolgt, nachdem der Druck in der genannten Kammer (21) jeweils auf einen im wesentlichen niedrigeren Druck als derjenige der Sterilisierung abgesunken ist.

2. Ein Verfahren gemäß Anspruch 1, wobei die Aktivierung des genannten zweiten Auslasskanals (11) vor der Deaktivierung des anderen Auslasskanals erfolgt.

3. Ein Verfahren gemäß Anspruch 1, wobei während mindestens einem Entleerungsschritt durch den genannten ersten Auslasskanal (10) Luftzirkulation durch den genannten zweiten Auslasskanal (11) stattfindet.

4. Ein Verfahren gemäß Anspruch 3, wobei jeweils nach dem genannten Luftzirkulationsschritt und vor einem Ablass-Schritt durch den genannten zweiten Auslasskanal (11) in dem genannten zweiten Auslasskanal (11) ein Vakuum gebildet wird.

5. Ein Verfahren gemäß Anspruch 1, wobei nach einem Sterilisierungsschritt ein Trocknungsschritt vorgesehen ist, bei dem intermittierend sterile Luft in die genannte Sterilisierungskammer (2a) eingelassen wird, während kontinuierlich Dampf aus der genannten Kammer (2a) gezogen wird.

6. Ein Verfahren gemäß Anspruch 3, wobei jeweils verschiedene Entleerungsschritte aus der genannten Sterilisierungskammer (2a) durch den genannten ersten Auslasskanal (10) vorgesehen sind, und mit jedem dieser genannten Schritte der genannte Luftzirkulationsschritt durch den genannten zweiten Auslasskanal (11) stattfindet.

7. Ein Verfahren gemäß Anspruch 1, wobei während mindestens einem Saugschritt durch den genannten zweiten Auslasskanal (11) eine intermittierende oder pulsierende Lufteinströmung zu dem genannten zweiten Auslasskanal (11) stattfindet, wobei die Entleerung der Sterilisierungskammer (2a) durch den genannten zweiten Auslasskanal (11) während jeder Pulsierungs- oder Lufteinströmungsperiode unterbrochen wird.

8. Ein Gerät zur Dampfsterilisierung, insbesondere für Verwendung im Dentalbereich, das einen Autoklav (2) umfasst, der eine Sterilisierungskammer (2a) bildet, einen Kanal (5) zur Versorgung der genannten Kammer (2a) mit Wasser, Elemente (3) zur Dampferzeugung, die mit dem genannten Autoklav (2) verbunden sind, einen Auslasskreis von der genannten Kammer (2a), der einen Kondensator (19) und mindestens ein Saugelement (21) sowie Steuerelemente zur Durchführung eines Sterilisierungszyklus umfasst, **dadurch gekennzeichnet, dass** der genannte Entleerungskreis mindestens einen ersten und einen zweiten Auslasskanal (10, 11) umfasst, die jeweils unabhängig voneinander und aufeinander folgend durch die genannten Steuerelemente aktiviert werden können, wobei der genannte Kondensator (19) und das genannte mindestens eine Saugelement (21) in dem genannten zweiten Auslasskanal (11) angeordnet sind.

9. Ein Gerät gemäß Anspruch 8, **dadurch gekennzeichnet, dass** ein Luftzufuhrkanal (23) vorgesehen ist, der mit dem genannten zweiten Auslasskanal (11) verbunden ist und gleichzeitig mit der Aktivierung des genannten ersten Auslasskanals (10) aktiviert werden kann.

10. Ein Gerät gemäß Anspruch 9, bei dem ein Hilfs-Auslasskanal (17) vorgesehen ist, der den genannten zweiten Auslasskanal (11) mit dem oberen Abschnitt der genannten Sterilisierungskammer (2a) verbindet, wobei der genannte Hilfskanal (17) jeweils hydraulisch von dem genannten zweiten Auslasskanal (11) getrennt werden kann.

11. Ein Gerät gemäß Anspruch 8, bei dem sich ausgehend vom Boden der genannten Sterilisierungskammer (2a) ein Abflusskanal (9) befindet, der sich in den genannten ersten Auslasskanal (10) und in den genannten zweiten Auslasskanal (11) verzweigt, wobei der genannte Abflusskanal (9) jeweils wahlweise hydraulisch mit dem genannten ersten Auslasskanal (10) und dem genannten zweiten Auslasskanal (11) verbunden werden kann.

12. Ein Gerät gemäß Anspruch 8, bei dem der genannte erste Auslasskanal (10) eine spulenförmige Länge (14) aufweist, die in dem genannten Speisetank (4) angeordnet ist, um einen wassergekühlten Kondensator (15) zu bilden.

13. Ein Gerät gemäß Anspruch 8, bei dem der genannte Kondensator (19), der in dem genannten zweiten Auslasskanal (11) angeordnet ist, ein luftgekühlter Kondensator ist.

14. Ein Gerät gemäß Anspruch 13, bei dem der genannte luftgekühlte Kondensator (19) aus einer Spule (18) besteht, die mit Kühlrippen (18a) ausgestattet ist.

15. Ein Gerät gemäß Anspruch 8, bei dem das genannte mindestens eine Saugelement (21) eine Vakuumpumpe ist, und zwar insbesondere eine Membranpumpe.

16. Ein Gerät gemäß Anspruch 8, bei dem mindestens ein weiterer Auslasskanal (30) vorgesehen ist, der aus der genannten Sterilisierungskammer (2a) herauskommt und mindestens teilweise gleichzeitig mit der Aktivierung des genannten ersten Auslasskanals (10) aktiviert werden kann.

## Revendications

1. Un procédé pour la stérilisation par la vapeur d'eau, en particulier pour des usages dentaires, dans lequel une chambre de stérilisation (2a) est amenée à la pression et à la température de stérilisation, et est ensuite évacuée en déchargeant le condensat et la vapeur d'eau de la chambre, et dans lequel un condensateur (19) et au moins un moyen d'aspiration (21) sont placés dans une conduite de décharge,
**caractérisé par le fait que** la décharge est réalisée à travers au moins une première et une seconde conduites de décharge (10,11) qui doivent être activées en succession, et **par le fait que** la décharge à travers ladite seconde conduite de décharge (11), équipée dudit condensateur (19) et dudit au moins un moyen d'aspiration (21), est réalisée après que la pression dans ladite chambre (21) soit tombée à une valeur substantiellement inférieure à la pression de stérilisation.

2. Un procédé conformément à la revendication 1, dans lequel l'activation de ladite seconde conduite de décharge (11) est réalisée avant la désactivation des autres conduites de décharge.

3. Un procédé conformément à la revendication 1, dans lequel durant au moins une phase de décharge à travers ladite première conduite de décharge (10), la circulation de l'air à travers ladite seconde conduite de décharge (11) est effectuée.

4. Un procédé conformément à la revendication 3, dans lequel après ladite phase de circulation de l'air et avant la phase de décharge qui a lieu à travers ladite seconde conduite de décharge (11), un vide est créé dans ladite seconde conduite de décharge (11).

5. Un procédé conformément à la revendication 1, dans lequel après une phase de stérilisation, une phase de séchage est réalisée dans laquelle de l'air stérile est admis dans ladite chambre de stérilisation (2a) d'une manière intermittente, alors que la vapeur d'eau est extraite de ladite chambre (2a) d'une manière continue.

6. Un procédé conformément à la revendication 3, dans lequel plusieurs phases de décharge depuis ladite chambre de stérilisation (2a) à travers ladite première conduite de décharge (10) sont réalisées, et à chacune desdites phases est associée ladite phase de circulation de l'air à travers ladite seconde conduite de décharge (11).

7. Un procédé conformément à la revendication 1, dans lequel durant au moins une phase d'aspiration à travers ladite seconde conduite de décharge (11), une admission d'air pulsé ou intermittent est réalisée dans ladite seconde conduite de décharge (11), l'évacuation de la chambre de stérilisation (2a) à travers ladite conduite de décharge (11) étant interrompue durant chaque période d'impulsion ou d'admission de l'air.

8. Un appareil pour la stérilisation par la vapeur d'eau, en particulier pour usages dentaires, comprenant un autoclave (2) définissant une chambre de stérilisation (2a), une conduite (5) pour l'alimentation de l'eau à ladite chambre (2a), des moyens (3) pour la génération de la vapeur d'eau associés audit autoclave (2), un circuit de décharge depuis ladite chambre (2a) comprenant un condensateur (19) et au moins un moyen d'aspiration (21) et des moyens de contrôle pour l'exécution d'un cycle de stérilisation,
**caractérisé par le fait que** ledit circuit de décharge comprend au moins une première et une seconde conduites de décharge (10, 11), à activer sélectivement d'une manière indépendante l'une de l'autre, et en succession par lesdits moyens de contrôle, ledit condensateur (19) et ledit au moins un moyen d'aspiration (21) étant placés dans ladite seconde conduite de décharge (11).

9. Un appareil conformément à la revendication 8, dans lequel est réalisée une conduite d'alimentation de l'air (23) qui est connectée à ladite seconde conduite de décharge (11) et qui peut être activée en même temps que l'activation de ladite première conduite de décharge (10).

10. Un appareil conformément à la revendication 9, dans lequel est réalisée une conduite de décharge auxiliaire (17) qui relie ladite seconde conduite de décharge (11) à la portion supérieure de ladite chambre de stérilisation (2a), ladite conduite auxiliaire (17) pouvant être séparée hydrauliquement de ladite seconde conduite de décharge (11).

11. Un appareil conformément à la revendication 8, dans lequel à partir du fond de ladite chambre de stérilisation (2a) se trouve une conduite de sortie (9) se ramifiant dans ladite première conduite de décharge (10) et dans ladite seconde conduite de décharge (11), ladite conduite de sortie (9) étant en mesure d'être connectée hydrauliquement de manière sélective à ladite première conduite de décharge (10) et à ladite seconde conduite de décharge (11), respectivement.

12. Un appareil conformément à la revendication 8, dans lequel la première conduite de décharge (10) a un tronçon en forme de spirale (14) placé dans ledit réservoir d'alimentation (4) de manière à former un condensateur (15) réfrigéré par l'eau.

13. Un appareil conformément à la revendication 8, dans lequel ledit condensateur (19) placé dans ladite seconde conduite de décharge (11) est un condensateur refroidi par l'air.

14. Un appareil conformément à la revendication 13, dans lequel ledit condensateur refroidi par l'air (19) se compose d'une spirale (18) dotée d'ailettes de refroidissement (18a).

15. Un appareil conformément à la revendication 8, dans lequel ledit au moins un moyen d'aspiration (21) est une pompe à vide, en particulier une pompe à diaphragme.

16. Un appareil conformément à la revendication 8, dans lequel est réalisée au moins une autre conduite de décharge (30) sortant de ladite chambre de stérilisation (2a) et qui peut être activée au moins en partie en même temps que l'activation de ladite première conduite de décharge (10).
